# EUROPEAN PATENT APPLICATION

(11) **EP 3 922 734 A1**
(43) Date of publication of application: **15.12.2021**
(21) Application number: 20179109.2
(22) Date of filing: 09.06.2020
(51) Int. Cl.: C12Q 1/6888

(54) **METHOD FOR DETERMINING PRESENCE OF A PRE-DETERMINED VIRAL RNA SEQUENCE IN A SAMPLE**

(71) Applicant: Ender diagnostics AG, 3018 Bern (CH)
(72) Inventor: Zürcher, Samuel, 3018 Bern (CH); Lüthi, Alexander, 3018 Bern (CH); Weibel, Lea, 3018 Bern (CH)
(74) Representative: Vossius & Partner Patentanwälte Rechtsanwälte mbB

(57) **Abstract**

The present invention relates to a method for determining presence of a pre-determined RNA sequence in a sample, the method comprising the steps of adding one or more enzyme(s) providing activities of RNA- and DNA-dependent DNA polymerase activity and strand-displacement activity to the sample to be analysed for the presence of the pre-determined RNA sequence; simultaneously or subsequently adding at least five DNA primers to the sample to be analysed for the presence of the pre-determined RNA sequence, wherein at least one DNA primer comprises a sequence hybridisable to the RNA sequence and at least one DNA primer comprises a sequence hybridisable to the DNA sequence reverse-complementary to the RNA sequence; incubating the sample resulting from the previous steps at a fixed temperature; and determining whether an elongated double-stranded DNA sequence is present in the sample, wherein presence of the elongated double-stranded DNA sequence in the sample is indicative of the presence of the pre-determined RNA sequence in the sample wherein the pre-determined RNA sequence is part of an RNA virus.

## Description

The present invention relates to a method for determining presence of a pre-determined RNA sequence in a sample, the method comprising the steps of adding one or more enzyme(s) providing activities of RNA- and DNA-dependent DNA polymerase activity and strand-displacement activity to the sample to be analysed for the presence of the pre-determined RNA sequence; simultaneously or subsequently adding at least five DNA primers to the sample to be analysed for the presence of the pre-determined RNA sequence, wherein at least one DNA primer comprises a sequence hybridisable to the RNA sequence and at least one DNA primer comprises a sequence hybridisable to the DNA sequence reverse-complementary to the RNA sequence; incubating the sample resulting from the previous steps at a fixed temperature; and determining whether an elongated double-stranded DNA sequence is present in the sample, wherein presence of the elongated double-stranded DNA sequence in the sample is indicative of the presence of the pre-determined RNA sequence in the sample wherein the pre-determined RNA sequence is part of an RNA virus.

There are various methods directed at detecting the presence of RNA sequences in samples. However, most of the known techniques require a substantial amount of time. Yet, in some applications, time is a limiting factor. For example, there are many coronaviruses in wild animal populations, but only a few developed the ability to infect humans, comprising SARS-CoV, MERS-CoV and the newly emerging SARS-CoV-2. All of them can cause severe respiratory, gastrointestinal, and central nervous system diseases.

In many cases, a coronavirus infection is not recognized by the patient or is recognized as a common cold. Moreover, the incubation time of coronaviruses before symptoms develop is about 4 days to 2 weeks. This makes it challenging for health-care systems to follow viral spread.

In addition, available molecular virus tests take several hours to provide a reliable result and have to be performed at a centralized laboratory. During this time, the potentially infected person may have already infected other persons, further spreading the virus.

In view of the above, there is an urgent need for fast and yet reliable tests for the presence of RNA, in particular viral RNA, more particular RNA derived from a corona virus, in a sample.

The above technical problem is solved by the embodiments provided herein and as characterized in the claims.

Accordingly, the present invention relates to the following embodiments.
1. A method for determining presence of a pre-determined RNA sequence in a sample, the method comprising the steps of:
   (a) adding one or more enzyme(s) providing activities of RNA- and DNA-dependent DNA polymerase activity and strand-displacement activity to the sample to be analysed for the presence of the pre-determined RNA sequence;
   (b) adding at least five DNA primers to the sample to be analysed for the presence of the pre-determined RNA sequence, wherein at least one DNA primer comprises a sequence hybridisable to the RNA sequence and at least one DNA primers comprise a sequence hybridisable to the DNA sequence reverse-complementary to the RNA sequence;
   (c) incubating the sample resulting from steps (a) and (b) at a fixed temperature;
   (d) determining whether an elongated double-stranded DNA sequence is present in the sample,
      wherein presence of the elongated double-stranded DNA sequence in the sample is indicative of the presence of the pre-determined RNA sequence in the sample
   wherein the pre-determined RNA sequence is part of an RNA virus.
2. The method of claim 1, wherein four of the at least five primers are forward inner primer (FIP), backward inner primer (BIP), loop primer forward (LPF) and loop primer backwards (LPB), respectively.
3. The method of claim 1 and 2, wherein the fifth primer is a B3 primer.
4. The method of any one of claims 1 to 3, wherein no F3 primer is used.
5. The method of any one of claims 1 to 4, wherein the enzyme having RNA-dependent DNA polymerase activity is a reverse transcriptase enzyme or a DNA polymerase with reverse transcriptase activity and, optionally, strand displacement activity.
6. The method of claim 1, wherein the RNA virus is a coronavirus.
7. The method of claim 6, wherein the coronavirus (CoV) is of the genus α-CoV, β-CoV, γ-CoV or δ-CoV.
8. The method of claim 6 or 7, wherein the coronavirus is selected from the group consisting of Human coronavirus OC43 (HCoV-OC43), Human coronavirus HKU1 (HCoV-HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019").
9. The method of any one of claims 1 to 8, wherein the fixed temperature is between 50 and 75°C.
10. The method of any one of claims 1 to 9, wherein the sample in step (c) is incubated for 1 to 120 minutes.
11. The method of any one of claims 1 to 10, wherein the virus is a corona virus and one or more of the primers comprise the following sequences:
   (a) FIP primer sequence: CTT GCT CTT CTT CAG GTT GAC CAA GGT AAA CCT TTG; and/or
   (b) BIP primer sequence: GGT TAG ATG ATG ATA GTC CTG ATT GTC CTC ACT GCC; and/or
   (c) LPF primer sequence: AAG AGC AGC AGA AGT GGC AC; and/or
   (d) LPB primer sequence: CAA ACT GTT GGT CAA CAA G; and/or
   (e) B3 primer sequence: GAA CCT CAA CAA TTG TTT GAA TAG.
12. The method of any one of claims 1 to 11, wherein presence of the elongated double-stranded DNA sequences in the sample is determined by using a nucleic acid molecule hybridisable to the elongated double-stranded DNA sequence, in particular wherein the nucleic acid molecule is labelled, or by using a molecule that intercalates in the elongated double-stranded DNA sequence or using turbidity measurement.
13. A method of treating a patient infected by a virus of the corona family, in particular Severe acute respiratory syndrome coronavirus 2 (SARS-CoV-2), the method comprising administering to the patient an efficient amount of antiviral treatment, wherein the patient has previously been determined to be infected by a virus of the corona family using the method of any one of claims 1 to 12.
14. The method of claim 13, wherein antiviral treatment comprises administering an efficient amount of Remdesivir to the patient.

Accordingly, in a first aspect, the invention relates to a method for determining presence of a pre-determined RNA sequence in a sample, the method comprising the steps of adding one or more enzyme(s) providing activities of RNA- and DNA-dependent DNA polymerase activity and strand-displacement activity to the sample to be analysed for the presence of the pre-determined RNA sequence; simultaneously or subsequently adding at least five DNA primers to the sample to be analysed for the presence of the pre-determined RNA sequence, wherein at least one DNA primer comprises a sequence hybridisable to the RNA sequence and at least one DNA primer comprises a sequence hybridisable to the DNA sequence reverse-complementary to the RNA sequence; incubating the sample resulting from the previous steps at a fixed temperature; and determining whether an elongated double-stranded DNA sequence is present in the sample, wherein presence of the elongated double-stranded DNA sequence in the sample is indicative of the presence of the pre-determined RNA sequence in the sample wherein the pre-determined RNA sequence is part of an RNA virus.

It is preferred within the present invention that five different primers are used.

It is preferred within the present invention that four of the at least five primers are forward inner primer (FIP), backward inner primer (BIP), loop primer forward (LPF) and loop primer backwards (LPB), respectively. These terms are commonly used in methods related to loop-mediated isothermal amplification (LAMP) methods, such as those described by Nagamine et al. 2002. Molecular and Cellular Probes 16. 223-229

However, within the present invention, it was surprisingly found that a five-primer system is most efficient in detecting a pre-determined RNA sequence. "Most efficient" as used herein means that detection is faster and more sensitive than commonly used techniques but maintains reliability, which is a prerequisite in tests used for detecting viral infection. In addition, it was found that by using five primers instead of six primers as in the standard LAMP technology, shorter target sequences can be detected.

Within the methods of the present invention, it is thus preferred that the fifth primer is a B3 primer. It is also preferred that no F3 primer is used. This is because it was surprisingly found by the inventors that in the presence of a B3 primer but absence of an F3 primer, detection is faster and more sensitive. Using the methods of the present invention, detection was observed to be possible within ten minutes and more sensitive to detect a low number of pre-determined RNA sequence in a sample. That is, as it is shown in the appended Examples, a positive detection of a pre-determined RNA sequence of a corona virus was achieved using five primers, in particular FIP, BIP, LPF, LPB and B3, within ten minutes after addition of primers and enzymes. Accordingly, the methods of the present invention, for the first time, provide a reliable and fast way to detect viral infections which is important in controlling a pandemic outbreak of the same.

Within the methods of the present invention, one or more enzyme(s) providing activities of RNA- and DNA-dependent DNA polymerase activity and strand-displacement activity are used. That is, all three activities are to be added to the RNA sequence to be analyzed. The activities can be provided by one enzyme having all three activities, or several enzymes each having one or more of the three activities.

Within the methods of the present invention, it is preferred that the RNA virus comprising the pre-determined RNA sequence is a coronavirus. However, the RNA virus may be of any kind. Within the present invention, the Coronavirus may in particular be of the genus α-CoV, β-CoV, γ-CoV or δ-CoV. More particularly, the Coronavirus may be selected from the group consisting of Human coronavirus OC43 (HCoV-OC43), Human coronavirus HKU1 (HCoV- HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019").

In the methods of the present invention, in particular in step (c) thereof, the temperature can be fixed. That is, the one or more enzyme(s), DNA primers and the sample to be analyzed are incubated in the same tube at a constant temperature. The temperature is preferably between 50 and 75°C. However, the temperature may also be lower, for example between 30 and 75°C. In an alternative embodiment, a touchdown temperature step is used. That is, the temperature is lowered during the course of the analysis, for example starting at a temperature of 70°C that is subsequently lowered to 50°C.

In the methods of the present invention, the one or more enzyme(s), DNA primers and the sample to be analyzed are incubated in the same tube for a time between 1 and 120 minutes, preferably between 1 and 60, 1 and 45, 1 and 30 or between 1 and 15 minutes. In a preferred embodiment, the sample is incubated for 3, 4, 5, 6, 7, 8, 9, 10, 11, 12, 13, 14, 15, 16, 17, 18, 19, 20, 21, 22, 23, 24, 25, 26, 27, 28, 29 or 30 minutes.

Within the present invention, it is preferred that the virus is a corona virus. In such an embodiment of the invention, the preferred primer sequences are shown below:
(a) FIP primer sequence: CTT GCT CTT CTT CAG GTT GAC CAA GGT AAA CCT TTG (SEQ ID NO: 1); and/or
(b) BIP primer sequence: GGT TAG ATG ATG ATA GTC CTG ATT GTC CTC ACT GCC (SEQ ID NO: 2); and/or
(c) LPF primer sequence: AAG AGC AGC AGA AGT GGC AC (SEQ ID NO: 3); and/or
(d) LPB primer sequence: CAA ACT GTT GGT CAA CAA G (SEQ ID NO: 4); and/or
(e) B3 primer sequence: GAA CCT CAA CAA TTG TTT GAA TAG (SEQ ID NO: 5).

The above primer sequences target a sequence of corona virus. In particular, the above primer target the following sequence

ACCAAGGTAAACCTTTGGAATTTGGTGCCACTTCTGCTGCTCTTCAACCTGAAGA AGAGCAAGAAGAAGATTGGTTAGATGATGATAGTCAACAAACTGTTGGTCAACA AGACGGCAGTGAGGACAATCAGACAACTACTATTCAAACAATTGTTGAGGTTC (SEQ ID NO: 7) corresponding to bases 3129-3290 of Accession no. NC_045512.2

However, the skilled person is well-aware how to design alternative or further primer sequences depending on the target sequence to be detected in the sample.

The methods of the present invention comprise a step of determining whether an elongated double-stranded DNA sequence is present in the sample, in particular wherein presence of the elongated double-stranded DNA sequence in the sample is indicative of the presence of the pre-determined RNA sequence in the sample. The skilled person is well-aware of methods suitable to be used for determining presence of an elongated double-stranded DNA sequence in a sample, in particular where the sequence to be detected is known. Thus, any method known to the skilled person for that purpose may be used within the present invention. However, it is preferred that the presence of the elongated double-stranded DNA is determined by using a molecule that intercalates in the elongated double-stranded DNA and thereby starts fluorescing which can be detected and quantified with specialised equipment. Other methods such as using a nucleic acid molecule hybridisable to the elongated double-stranded DNA sequence, in particular wherein the nucleic acid molecule is labelled or bound to a surface, or using turbidity measurement of the enzyme reaction can be used to determine the elongation of the DNA.

The present invention further relates to a method of treating a patient infected by a virus of the corona family, in particular Severe acute respiratory syndrome coronavirus 2 (SARS-CoV-2), the method comprising administering to the patient an efficient amount of antiviral treatment, wherein the patient has previously been determined to be infected by a virus of the corona family using the method of the present invention. It is preferred that antiviral treatment comprises administering an efficient amount of Remdesivir to the patient.

In a further embodiment, the invention relates to a kit, in particular a kit for use in detecting viral RNA in a sample. The kit comprises one or more, preferably all five or six primers for detecting a pre-determined RNA sequence. The kit may also comprise more than one primer system, in particular two or more primer systems targeting different sequences of the same RNA virus.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While aspects of the invention are illustrated and described in detail in the figures and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

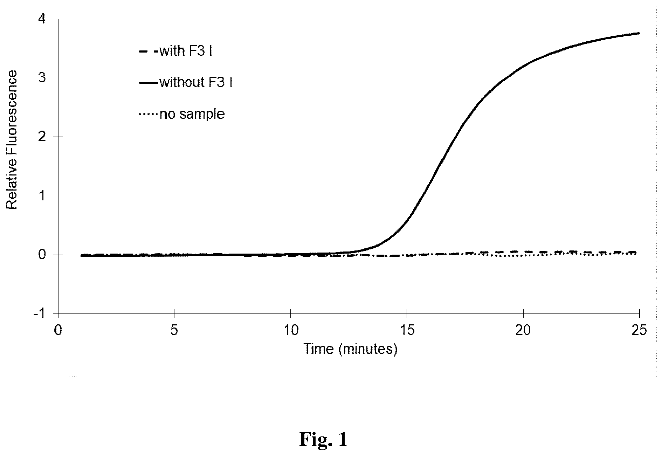

**Figure 1** shows the sensitivity compared between the 5 primers system of the invention and the 6 primer system on a sample with a low copy number of SARS-CoV-2 RNA .

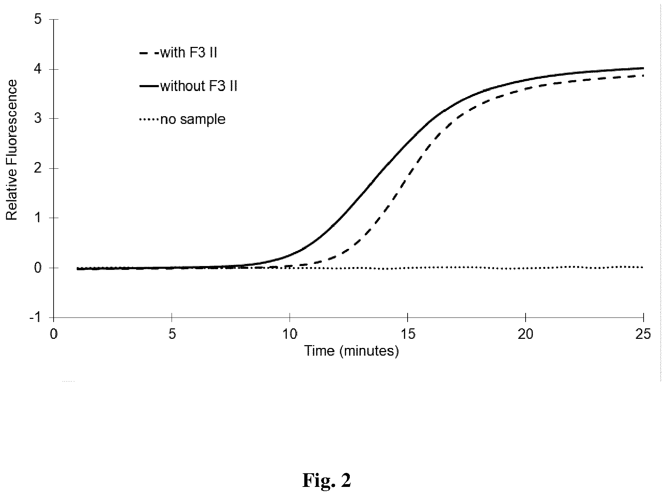

**Figure 2** shows the time after which signal is determined using the 5 primer system and the 6 primer system.

### Examples

The following are examples of methods and compositions of the invention. It is understood that various other embodiments may be practiced, given the general description provided above.

**The novel 5 primer system without F3 amplifies SARS-CoV2 RNA as efficient as 6 primer system with F3**

**Table 1 - Primers**

| | | | |
|---|---|---|---|
| FIP | CTT GCT CTT CTT CAG GTT GAC CAA GGT AAA CCT TTG (SEQ ID NO: 1) | LPF | AAG AG C A GC AGA AG T G GC AC (SEQ ID NO: 3) |
| BIP | GGT TAG ATG ATG ATA GTC CTG ATT GTC CTC ACT GCC (SEQ ID NO: 2) | LPB | CAA ACT GTT GGT CAA CAA G (SEQ ID NO: 4) |
| B3 | GAA CCT CAA CAA TTG TTT GAA TAG (SEQ ID NO: 5) | F3 | GCC ATC AAC TCA ATA TGA GTA TG (SEQ ID NO: 6) |

**Table 2 - Primer mix: novel 5 primer system**

| | **Final concentration.** |
|---|---|
| FIP | 1.6 µM |
| BIP | 1.6 µM |
| LPF | 0.8 µM |
| LPB | 0.8 µM |
| B3 | 0.4 µM |

**Table 3 - Primer mix: LAMP 6 primer system**

| | **Final concentration** |
|---|---|
| FIP | 1.6 µM |
| BIP | 1.6 µM |
| LPF | 0.8 µM |
| LPB | 0.8 µM |
| B3 | 0.2 µM |
| F3 | 0.2 µM |

**Table 4 - Primer/Enzyme mix (PEM)**

| | **Vol/rx** |
|---|---|
| **Isothermal master mix** | **15.0 µl** |
| **Primer mix** | **2.0 µl** |
| | **17.0 µl** |

| | |
|---|---|
| Add 17.0 µl PEM per reaction | |

### Template addition

Add 8.0 µl extracted RNA

Add 8.0 µl RNase-free H2O as negative assay control

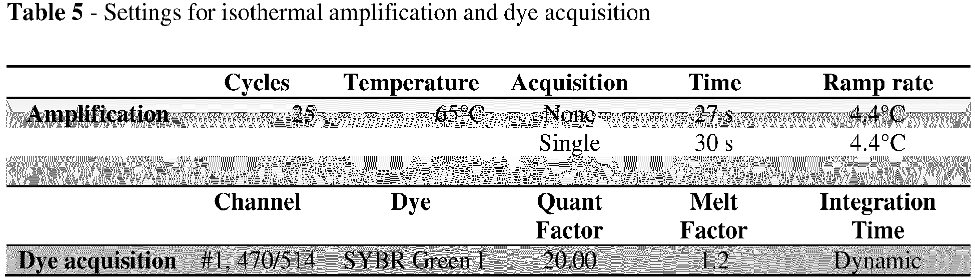

## Claims

1. A method for determining presence of a pre-determined RNA sequence in a sample, the method comprising the steps of:
(a) adding one or more enzyme(s) providing activities of RNA- and DNA-dependent DNA polymerase activity and strand-displacement activity to the sample to be analysed for the presence of the pre-determined RNA sequence;
(b) simultaneously or subsequently to step (a), adding at least five DNA primers to the sample to be analysed for the presence of the pre-determined RNA sequence, wherein at least one DNA primer comprises a sequence hybridisable to the RNA sequence and at least one DNA primer comprises a sequence hybridisable to the DNA sequence reverse-complementary to the RNA sequence;
(c) incubating the sample resulting from steps (a) and (b) at a fixed temperature;
(d) determining whether an elongated double-stranded DNA sequence is present in the sample,
wherein presence of the elongated double-stranded DNA sequence in the sample is indicative of the presence of the pre-determined RNA sequence in the sample
wherein the pre-determined RNA sequence is part of an RNA virus.

2. The method of claim 1, wherein four of the at least five primers are forward inner primer (FIP), backward inner primer (BIP), loop primer forward (LPF) and loop primer backwards (LPB), respectively.

3. The method of claim 1 and 2, wherein the fifth primer is a B3 primer.

4. The method of any one of claims 1 to 3, wherein no F3 primer is used.

5. The method of any one of claims 1 to 4, wherein the enzyme having RNA-dependent DNA polymerase activity is a reverse transcriptase enzyme and/or a DNA polymerase with reverse transcriptase activity.

6. The method of claim 1, wherein the RNA virus is a coronavirus.

7. The method of claim 6, wherein the coronavirus is of the genus α-CoV, β-CoV, γ-CoV or δ-CoV.

8. The method of claim 6 or 7, wherein the coronavirus is selected from the group consisting of Human coronavirus OC43 (HCoV-OC43), Human coronavirus HKU1 (HCoV-HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019").

9. The method of any one of claims 1 to 8, wherein the fixed temperature is between 50 and 75°C.

10. The method of any one of claims 1 to 9, wherein the sample in step (c) is incubated for 1 to 120 minutes.

11. The method of any one of claims 1 to 10, wherein the virus is a corona virus and one or more of the primers comprise the following sequences:
(f) FIP primer sequence: CTT GCT CTT CTT CAG GTT GAC CAA GGT AAA CCT TTG; and/or
(g) BIP primer sequence: GGT TAG ATG ATG ATA GTC CTG ATT GTC CTC ACT GCC; and/or
(h) LPF primer sequence: AAG AGC AGC AGA AGT GGC AC; and/or
(i) LPB primer sequence: CAA ACT GTT GGT CAA CAA G; and/or
(j) B3 primer sequence: GAA CCT CAA CAA TTG TTT GAA TAG.

12. The method of any one of claims 1 to 11, wherein presence of the elongated double-stranded DNA sequences in the sample is determined by using a nucleic acid molecule hybridsable to the elongated double-stranded DNA sequence, in particular wherein the nucleic acid molecule is labelled, using a molecule that intercalates in the elongated double-stranded DNA sequence or using turbidity measurement.

13. A method of treating a patient infected by a virus of the corona family, in particular Severe acute respiratory syndrome coronavirus 2 (SARS-CoV-2), the method comprising administering to the patient an efficient amount of antiviral treatment, wherein the patient has previously been determined to be infected by a virus of the corona family using the method of any one of claims 1 to 12.

14. The method of claim 13, wherein antiviral treatment comprises administering an efficient amount of Remdesivir to the patient.


SEQUENCE LISTING

<110> Ender diagnostics AG

<120> Method for determining presence of a pre-determined RNA sequence in a
sample

<130> AB1828 EP BS

<160> 7

<170> BiSSAP 1.3.6

<210> 1
<211> 36
<212> DNA
<213> Artificial Sequence

<220>
<223> FIP primer

<400> 1
cttgctcttc ttcaggttga ccaaggtaaa cctttg                                36

<210> 2
<211> 36
<212> DNA
<213> Artificial Sequence

<220>
<223> BIP primer

<400> 2
ggttagatga tgatagtcct gattgtcctc actgcc                                36

<210> 3
<211> 20
<212> DNA
<213> Artificial Sequence

<220>
<223> LPF primer

<400> 3
aagagcagca gaagtggcac                                                  20

<210> 4
<211> 19
<212> DNA
<213> Artificial Sequence

<220>
<223> LPB primer

<400> 4
caaactgttg gtcaacaag                                                   19

<210> 5
<211> 24
<212> DNA
<213> Artificial Sequence

<220>
<223> B3 primer

<400> 5
gaacctcaac aattgtttga atag                                              24

<210> 6
<211> 23
<212> DNA
<213> Artificial Sequence

<220>
<223> F3 primer

<400> 6
gccatcaact caatatgagt atg                                               23

<210> 7
<211> 162
<212> RNA
<213> Coronaviridae

<220>
<223> Bases 3129-3290 of Accession no. NC_045512.2 of SARS-CoV-2

<400> 7
accaaggtaa acctttggaa tttggtgcca cttctgctgc tcttcaacct gaagaagagc    60

aagaagaaga ttggttagat gatgatagtc aacaaactgt tggtcaacaa gacggcagtg   120

aggacaatca gacaactact attcaaacaa ttgttgaggt tc                      162